# EUROPÄISCHE PATENTSCHRIFT

(11) **EP 4 065 190 B1**
(45) Veröffentlichungstag und Bekanntmachung des Hinweises auf die Patenterteilung: **10.06.2026**
(21) Anmeldenummer: 20812297.8
(22) Anmeldetag: 25.11.2020
(51) Int. Cl.: A61M 1/16

(54) **VENTILINSEL FÜR EINE HYDRAULIKANORDNUNG FÜR EINE DIALYSEMASCHINE**
VALVE ISLAND FOR A HYDRAULIC ARRANGEMENT FOR A DIALYSIS MACHINE
ÎLOT DE SOUPAPES POUR UN ARRANGEMENT HYDRAULIQUE POUR UNE MACHINE DE DIALYSE

(30) Priorität: 27.11.2019 DE 102019132178
(43) Veröffentlichungstag der Anmeldung: 05.10.2022
(73) Patentinhaber: B. Braun Avitum AG, 34212 Melsungen (DE)
(72) Erfinder: BERTRAM, Rolf, 34286 Bergheim (DE); MÄNZ, Peter, 34212 Melsungen (DE)
(74) Vertreter: Winter, Brandl - Partnerschaft mbB
(86) Internationale Anmeldenummer: PCT/EP2020/083282
(87) Internationale Veröffentlichungsnummer: WO 2021/105158

(56) Entgegenhaltungen:
- EP-B1- 1 509 261
- WO-A1-2014/090746
- CN-A- 104 454 113

## Beschreibung

### Technisches Gebiet

Die vorliegende Offenbarung betrifft eine Ventilinsel für eine Hydraulikanordnung in einer Dialysemaschine, eine Hydraulikanordnung mit Ventilinsel sowie eine Dialysemaschine mit einer entsprechenden Hydraulikanordnung, welche die Ventilinsel aufweist.

### Stand der Technik

Dialysevorrichtungen/Dialysemaschinen in der Medizintechnik beinhalten in der Regel ein Hydrauliksystem bzw. eine Hydraulikanordnung, in dem Ventileinheiten eingesetzt werden, in denen jeweils (elektromagnetisch geschaltete) Ventile die Ein- und Ausgänge von Fluidleitungskomponenten der Dialysemaschine wahlweise sperren oder freigeben können, um einen gewünschten Fluidströmungspfad auszubilden.

Konventionelle Ventileinheiten für Dialysemaschinen wie zum Beispiel gemäß der Patentschrift EP 895787 setzen hierfür einen Grundaufbau ein, bei dem in diesem Fall eine Bilanziereinrichtung der Dialysemaschine über Magnetventile mit dem Hydrauliksystem verbunden ist. Die Magnetventile dienen hierbei als Sperrventile (Auf- /Zu-Ventile) für die Ein- und Ausgänge der Bilanzkammern der Bilanziereinrichtung. Über eine zentrale Steuerungseinheit werden die Magnetventile zum Ausbilden eines gewünschten Fluidströmungspfads angesteuert. Ferner, beschreibt das Dokument WO 2014 / 090 746 A1 eine Dialysemaschine mit einer Hydraulikanordnung, die eine durch Spritzgießen gefertigte Ventilinsel aufweist.

Nachteil dieser Lösung ist allerdings, dass die Ventileinheit viele einzelne Komponenten aufweist und die Anordnung eine dementsprechend große Einbaugröße erreichen kann.

Bei anderen Lösungen in der Medizintechnik werden bei Ventileinheiten dieser Gattung zentrale Montagebleche verwendet, auf denen Magnetventile befestigt sind, um einen zentralen Abschnitt bzw. eine Ventilinsel in dem Hydrauliksystem zu bilden. Gemäß einem Stand der Technik wird beispielsweise ein Einschubblech verwendet, auf dem das (die) Magnetventil(e) mittels einer Schraubverbindung befestigt ist (sind).

Der Nachteil dieser Lösungen ist jedoch, dass für Montage und Reparatur stets Werkzeuge benötigt werden, um die Ventile zu befestigen oder die Verbindung der Ventile zu lösen. Das führt zu einer Vergrößerung des benötigten Einbauraums sowie eine Erhöhung der Montageschritte.

### Kurzbeschreibung der Offenbarung

Die Aufgabe der Offenbarung ist es deshalb, eine Ventilinsel für eine gattungsgemäße Hydraulikanordnung/Hydrauliksystem und eine Hydraulikanordnung/Hydrauliksystem mit Ventilinsel insbesondere einer oder für eine Dialysemaschine bereitzustellen, mit denen der Montageaufwand bei einer gleichzeitigen Verringerung der Einbaugröße der Ventilinsel reduziert werden kann.

Diese Aufgabe wird mit einer Ventilinsel für eine Hydraulikanordnung (Hydrauliksystem) nach Anspruch 1, mit einer Hydraulikanordnung (Hydrauliksystem) mit Ventilinsel nach Anspruch 14 sowie einer Dialysemaschine mit einer, die Ventilinsel aufweisenden Hydraulikanordnung (Hydrauliksystem) nach Anspruch 19 gelöst.

Der Kern der vorliegenden Offenbarung besteht demzufolge grundsätzlich in der Anordnung einer Ventilinsel mit einem Grundkörper, an welchem zumindest eine Fluidleitung (Röhre/Fluidströmungskanal) in Form eines Rohres integral/stoffeinstückig ausgeformt ist, an deren zumindest einem, vorzugsweise beiden axialen Enden (stirnseitig) jeweils eine hydraulische/pneumatische Komponente, insbesondere ein Ventil und weiter vorzugsweise ein Magnetventil angeordnet/montiert ist, über das eine Fluidverbindung zwischen einer jeweiligen (vorzugsweise end-/stirnseitigen) Fluidleitungsöffnung und einer/einem Komponenten-/Ventil-seitigen Zweigleitung/-kanal geöffnet und/oder geschlossen werden kann. Die integrale, bzw. stoffeinstückig mit dem Grundkörper ausgebildete Fluidleitung hat ferner eine Anzahl (mindestens ein oder mehrere) von in Axialrichtung der Fluidleitung beabstandeter sowie ggf. umfangsversetzt angeordneter Abzweigungsanschlüsse (Tüllen), die ebenfalls stoffeinstückig mit dem Grundkörper ausgeformt sind. Vorzugsweise sind die Tüllen voneinander fluidgetrennt (z.B. durch eine Trennwand in der integralen Fluidleitung), derart, dass jeweils eine Tülle nur einem stirnseitig angeordneten (Magnet-)Ventil zugeordnet ist, sodass die zwei axial endseitig angeordneten (Magnet-)Ventile quasi mediengetrennt sind. Des Weiteren kann an der integralen Fluidleitung ein weiterer Port beispielsweise für den optionalen Anschluss eines zusätzlichen (Magnet-)Ventils stoffeinstückig angeformt sein. Schließlich können am Grundkörper eine Anzahl (mindestens ein) von Montage-/Haltemitteln (Vorsprünge, Haken, Laschen, etc.) stoffeinstückig an-/ausgeformt sein, welche zur Befestigung des Grundkörpers an einer separaten (nicht zur Ventilinsel zugehörigen) Halterung/Plattform vorgesehen und ausgebildet sind.

Durch diesen konstruktiven Aufbau der offenbarungsgemäßen Ventilinsel ist es möglich, deren Grundkörper (einschließlich sämtlicher daran einstückig vorgesehener Bestandteile gemäß vorstehender Ausführung) einteilig/stoffeinstückig im Rahmen eines einzigen Fertigungsschritts, insbesondere nach einem Spritzguss- oder 3D-Druckverfahren (rapid prototyping process) herzustellen. Die dabei (gleichzeitig) mitausgebildeten Anschlüsse an der integralen Fluidleitung erlauben (nachträglich) eine Montage von (Magnet-)Ventilen, externen Fluidleitungen, Temperatur-/elektrische Leitfähigkeits-/Druck-/Fluidströmungssensoren und dergleichen hydraulischen/pneumatischen Komponenten ohne den Einsatz von Werkzeug. Die Herstellung der so gestalteten Ventilinsel ist demzufolge preisgünstig und einfach. Da die Montagemittel bevorzugt ebenfalls am Grundkörper integriert sind, geht der Einbau der Ventilinsel in eine Maschine, vorzugsweise Dialysemaschine schnell vonstatten.

Gemäß der Erfindung ist die Ventilinsel für eine Hydraulikanordnung für eine oder einer extrakorporalen Blutbehandlungsmaschine, vorzugsweise Dialysemaschine vorgesehen. Die Ventilinsel weist den Grundkörper auf, in dem zumindest der eine, insbesondere starre, Fluidströmungskanal (integrale Fluidleitung) ausgebildet ist. Der Grundkörper ist, wie vorstehend ausgeführt wurde, durch additive Fertigung, oder Spritzgießen gefertigt

Durch Verwendung von 3D-Druck- oder Spritzgussverfahren ist die Herstellung des Grundkörpers in einem Schritt realisierbar. Dabei können der eine oder die mehreren integralen Fluidströmungskanäle variabel ausgestaltet werden. Beim Spritzgießen können hierfür Wechseleinsätze zum Einsatz kommen. Der zumindest eine integrale Fluidströmungskanal ist bevorzugt starr vorgesehen und kann daher auch eine tragende Funktion übernehmen. Aufgrund dieser selbsttragenden Funktion kann bei der Ventilinsel oder der Hydraulikanordnung auf ein konventionelles Gehäuse verzichtet werden kann.

In einer Weiterbildung der Ventilinsel ist (stoffeinstückig mit dem Grundkörper) zumindest ein Ventil-Montageabschnitt ausgebildet/angeordnet, der, wie vorstehend ausgeführt wurde, an zumindest einem axialen Ende/Endabschnitt der integralen Fluidleitung (Fluidströmungskanal) vorgesehen ist. Der Ventil-Montageabschnitt kann beispielsweise als ein Spulenelement, insbesondere ein Spulenträger für ein Magnetventil, ein Magnetventil selbst, ein Ventilsitz, eine Steuerkante, ein einfaches Schraubgewinde oder Bajonettverschluss oder eine andere Art eines Ventil-Montageabschnitts ausgebildet sein.

An dem Grundkörper kann der Ventil-Montageabschnitt oder das Magnetventil auch lösbar / verdrehbar angeordnet sein, wobei der Ventil-Montageabschnitt bevorzugt additiv oder weiter bevorzugt mittels Spritzgießen gefertigt ist. Unterschiedliche Varianten des Magnetventils, des Ventilsitzes usw., können mit einem SpritzgießWerkzeug gefertigt sein. Insbesondere ein stoffeinstückig am Grundkörper ausgebildeter Ventil-Montageabschnitt (beispielsweise in Form des zuvor beschriebenen Spulenaufnahmekörpers) reduziert die Anzahl von Einzelteilen, die an dem Grundkörper befestigt werden müssen. Mit anderen Worten reduziert sich die Anzahl der Einzelteile bzw. benötigter Komponenten. Darüber hinaus spart man die dafür erforderlichen Montagezeiten ein. Des Weiteren kann der hierfür vorgesehene Einbauraum, der für eine gesamte Hydraulikanordnung eingeplant werden muss, effizient reduziert werden.

Der Ventil-Montageabschnitt kann zusammen mit einem Ventilsitz angeordnet sein und die Funktion eines mediengetrennten Ventils erfüllen, um eine Kontaminationssicherheit des extrakorporalen Fluidkreislaufs zu gewährleisten.

In einer Weiterbildung der Ventilinsel ist das zumindest eine, stirnseitig am integralen Fluidströmungskanal angeordnete Magnetventil in Schritten insbesondere von kleiner oder gleich 45° axial drehbar am Grundkörper gehalten. Damit kann die Kabelverlegung für den Ventilabschnitt individuell angepasst oder ausgerichtet und auch in Bezug auf Materialbedarf und Zugänglichkeit im Montage- und Servicefall optimiert werden. Das Ventil ist dabei vorzugsweise mechanisch gesichert, um ein selbsttätiges Verdrehen zu vermeiden. Zusätzlich kann Kabellänge eingespart werden.

In einer Weiterbildung der Ventilinsel ist einstückig mit dem Grundkörper zumindest die eine, insbesondere hohlzylinderförmige, Kopplungsaufnahme (vorstehend als Port bezeichnet) ausgebildet, über die eine (weitere) hydraulische Komponente, insbesondere radialdichtend/radialdicht, mit dem zumindest einen Fluidströmungskanal fluidisch und formschlüssig verbindbar, insbesondere verbunden, ist. Die Kopplungsaufnahme kann an den Grundkörper mittels Spritzgießen angespritzt oder mittels 3D-Druck angedruckt sein. Eine radialdichte Adaption von anderen hydraulischen Komponenten an dem Grundkörper wie zum Beispiel einem (weiteren) Ventil, einer Schlauchkupplung, eines Leitfähigkeitsmesssensors, eines Druckmesssensors, eines Temperatursensors, eines Druckreglers, einer Drossel, einem Druckminderungsventil oder dergleichen, ist dadurch ermöglicht.

In einer Weiterbildung der Ventilinsel weist die insbesondere hohlzylinderförmige Kopplungsaufnahme (Port) umfangsseitig, insbesondere innenumfangsseitig, zumindest eine, vorzugsweise eine Mehrzahl, von Vertiefungen (Längsnuten) auf, in die ein dafür angepasstes Element einer hydraulischen Komponente einsetzbar, insbesondere eingesetzt, ist. In anderen Worten ausgedrückt hat die Kopplungsaufnahme einen mit einem bestimmten Profil ausgebildeten Anschlussabschnitt, in/auf welchen die hydraulische Komponente (weiteres Ventil) drehfest sowie ggf. in einer vorbestimmten Drehposition bezüglich des Anschlussabschnitts ein-/aufgesetzt werden kann. Dadurch kann eine Drehung der hydraulischen Elemente relativ zu der Kopplungsaufnahme vermieden werden. Eine derartige Verdrehsicherung, beispielsweise durch Verzahnung (Nuten) ermöglicht eine verbesserte Ausrichtung der hydraulischen Komponente in Hinsicht auf die Verlegung der hydraulischen Leitungen, insbesondere Schläuche, und der Verlegung der Kabel oder des Kabelbaums der Dialysemaschine. Die Verdrehsicherung kann alternativ unterschiedliche Geometrien mit unterschiedlichen Abmaßen aufweisen. Damit kann ein Fehlerverhinderungsprinzip, wie zum Beispiel das "Poka-Yoke-Prinzip" realisiert werden. In einem Fall kann zum Beispiel nur eine (einzige) Ausrichtung der Komponenten möglich sein. Für ein axiales Sichern des hydraulischen Elements in der Kopplungsaufnahme kann bevorzugt ein Clip-förmiger Splint vorgesehen sein, der quer zur Kopplungsaufnahme in diese einsteckbar ist und somit ein axiales Ab-/Herausziehen des hydraulischen Elements aus/von der Kopplungsaufnahme verhindert.

In einer Weiterbildung der Ventilinsel ist stoffeinstückig mit dem Grundkörper zumindest eine Tülle, ausgebildet, über die eine externe fluidische Leitung mit dem zumindest einen integralen Fluidströmungskanal fluidisch verbindbar, insbesondere verbunden, ist. Die zumindest eine Tülle kann an den Grundkörper mittels Spritzgießen angespritzt oder mittels 3D-Druck angedruckt sein.

Der Durchmesser der zumindest eine Tülle kann variabel gestaltet sein. Die zumindest eine Tülle ist für das Verbinden bzw. Aufstecken von fluidischen Leitungen mit einem Innendurchmesser von insbesondere 2 bis 6mm vorgesehen. Die fluidische Leitung ist insbesondere ein Schlauch, kann aber alternativ ein Rohr sein. Der Grundkörper weist vorzugsweise 2 bis 3 oder 4 bis 6 Tüllen auf. Die Anzahl der Tüllen ist auf maximal 7 erweiterbar.

In einer Weiterbildung der Ventilinsel ist stoffeinstückig mit dem Grundkörper zumindest ein fluidischer und ggf. auch mechanischer Verbindungsabschnitt ausgebildet, über den eine vorzugsweise sensorische Komponente, insbesondere eine hydraulische Sensorkomponente, fluidisch und ggf. auch mechanisch anbindbar, insbesondere angebunden, ist. Eine radialdichte/radialdichtende Adaption oder Anbindung von Sensorkomponenten an dem Grundkörper, wie zum Beispiel ein Leitfähigkeitsmesssensor, einem Druckmesssensor, einem Temperatursensor oder dergleichen wird dadurch ermöglicht. Alternativ können andere hydraulische Komponenten wie zum Beispiel ein (weiteres) Ventil, eine Schlauchkupplung, ein Druckregler, eine Drossel, ein Druckminderungsventil, oder dergleichen angebunden werden.

In einer Weiterbildung der Ventilinsel ist am Grundkörper zumindest ein Verbindungsabschnitt ausgebildet, über den die Ventilinsel mit einem darauf abgestimmten Verbindungsabschnitt der Hydraulikanordnung oder eines Montageadapters verbindbar, insbesondere verbunden, ist.

In einer Weiterbildung der Ventilinsel sind am Grundkörper zumindest zwei Ausnehmungen, insbesondere zwei Bohrungen ausgebildet, durch die Fixiermittel, insbesondere Kabelbinder, durchführbar, insbesondere durchgeführt sind. Alternativ können am Grundkörper auch eine andere Anzahl an Bohrungen beispielsweise vier Bohrungen ausgebildet/vorgesehen sein, um eine den entsprechenden Umständen angepasste Fixierung zu ermöglichen. Die Fixiermittel sollen eine Zugentlastung für zumindest ein Kabel oder einen Kabelstrang ermöglichen.

In einer Weiterbildung der Ventilinsel ist im Grundkörper zumindest eine Aufnahme, insbesondere ein Steckerfenster, ausgebildet, in der ein Steckergehäuse aufnehmbar, insbesondere aufgenommen, ist. Alternativ können am Grundkörper zwei Steckerfenster ausgebildet sein, die je ein Steckergehäuse aufnehmen. Das Steckerfenster ermöglicht die Aufnahme eines Steckergehäuses eines Laststrangs oder eines Steckergehäuses eines Sensorstrangs.

In einer Weiterbildung der Ventilinsel ist die hydraulische Komponente ein Ventil, insbesondere ein Magnetventil, das über die endseitig am integrierten Fluidströmungskanal angeordnete/ausgebildete Kopplungsaufnahme mit dem zumindest einen integrierten Fluidströmungskanal, insbesondere radialdicht, fluidisch und formschlüssig (stirnseitig) verbunden ist. In einer Weiterbildung der Ventilinsel weist das Ventil einen Ventilsitz auf, der insbesondere durch additive Fertigung oder Spritzgießen gefertigt ist.

In einer Weiterbildung der Ventilinsel weist das Ventil einen, insbesondere kegelstumpfförmigen, Kopplungsabschnitt auf, der in die vorstehend beschriebene Kopplungsaufnahme eingesetzt ist, und darin über ein Montageelement, insbesondere einem Montageclip (Splint gemäß vorstehender Definition), axial befestigt ist. Der Kopplungsabschnitt kann durch Spritzgießen oder additive Fertigung gefertigt sein. Durch den Einsatz des kegelstumpfförmigen Kopplungsabschnitts in die Kopplungsaufnahme wird eine fluidische, radialdichte Verbindung hergestellt. Mittels der fluidischen, radialdichten Verbindung werden die Fluidströmungskanäle des Grundkörpers und des Ventils verbunden. Das Montageelement (Splint) kann mittels Spritzgießen oder additiver Fertigung gefertigt sein oder als Stanzbiegeteil oder als Drahtbiegeteil hergestellt sein.

In einer Weiterbildung der Ventilinsel weist der vorstehend genannte Kopplungsabschnitt ein für die zumindest eine Vertiefung der Kopplungsaufnahme angepasstes Element, insbesondere einen Vorsprung, auf. Der Vorsprung des Kopplungsabschnitts greift in die Vertiefung der Kopplungsaufnahme. Damit wird eine Verdrehsicherung des Ventilabschnitts im Verhältnis zum Grundkörpers realisiert.

In einer Weiterbildung der Ventilinsel weist der Kopplungsabschnitt an einem Abschnitt insbesondere am Umfang eines Spitzenendes ein Dichtelement, insbesondere einen O-Ring, zum Abdichten eines gebildeten Fluidströmungskanals auf.

In einer Weiterbildung der Ventilinsel weist das Ventil einen Ventilabschnitt, insbesondere ein Spulenelement (Spulenaufnahmegehäuse), auf. Der Ventilabschnitt kann im Verhältnis zum Kopplungsabschnitt axial oder abgewinkelt, beispielsweise um etwa 90° abgewinkelt ausgebildet sein.

In einer Weiterbildung der Ventilinsel ist der Ventilabschnitt in Schritten, insbesondere von kleiner oder gleich 45°, axial drehbar angeordnet.

In einer Weiterbildung der Ventilinsel weist das Ventil zumindest eine Tülle (Ventilseitiger Zweigkanal gemäß vorstehender Definition) auf, über die eine fluidische externe Leitung, insbesondere ein Rohr oder ein Schlauch, mit dem zumindest einen integralen Fluidströmungskanal fluidisch verbindbar, insbesondere verbunden, ist.

Die zumindest eine Tülle kann im Verhältnis zum Kopplungsabschnitt und/oder dem Ventilabschnitt axial oder abgewinkelt, beispielsweise um etwa 90° abgewinkelt ausgebildet sein.

Das Ventil kann zwei oder mehrere Tüllen aufweisen, die zueinander axial oder abgewinkelt, beispielsweise um etwa 90° abgewinkelt ausgebildet sind.

In einer alternativen Weiterbildung der Ventilinsel weist das Ventil einen fluidischen und ggf. auch mechanischen Verbindungsabschnitt auf, über den eine insbesondere hydraulische Komponente fluidisch und ggf. auch mechanisch an dem Ventil anbindbar, insbesondere angebunden, ist.

Beispiele einer hydraulischen Komponente sind zum Beispiel ein weiteres Ventil, insbesondere Steckventil, eine Schlauchkupplung, ein Leitfähigkeitsmesssensor, ein Druckmesssensor, ein Temperatursensor, ein Druckregler, eine Drossel, ein Druckminderungsventil oder dergleichen sensorische Komponente,

Der fluidische und ggf. auch mechanische Verbindungsabschnitt kann im Verhältnis zum Kopplungsabschnitt und/oder dem Ventilabschnitt und/oder der zumindest einen Tülle axial oder abgewinkelt, beispielsweise um etwa 90° abgewinkelt, ausgebildet sein.

In einer Weiterbildung der Ventilinsel ist ein Mischelement zum Mischen von Fluiden in einem Fluidströmungskanal des/eines Ventils integriert. Das Ventil ist vorzugsweise ein hydraulisches Rückschlagventil oder ein hydraulisches Magnetventil.

In einer alternativen Weiterbildung der Ventilinsel ist ein Mischelement in einem Ventilsitz des Ventils integriert. Das Ventil ist vorzugsweise ein hydraulisches Magnetventil, insbesondere das Steckventil.

In einer Weiterbildung der Ventilinsel ist das Mischelement rohrförmig und erstreckt sich seiner Länge nach in Axialrichtung innerhalb des Fluidströmungskanal des Ventils.

In einer Weiterbildung der Ventilinsel ist die hydraulische Komponente ein Mischelement, das über die/eine Kopplungsaufnahme einem Fluidströmungskanal oder dem zumindest einen Fluidströmungskanal fluidisch und formschlüssig verbunden ist.

In einer Weiterbildung der Ventilinsel, in der die hydraulische Komponente ein Mischelement ist, hat das Mischelement vorzugsweise eine Vollzylinderform. Innerhalb des vollzylinderförmigen Mischelements sind eine Vielzahl von Strömungskanälen in der Erstreckungsrichtung des Mischelements ausgebildet. Die Strömungskanäle sind parallel zueinander und parallel zu einer Mittelachse des Mischelements angeordnet, um einen multiplen Durchgang für ein durchströmendes Fluid zu bilden. Die Strömungskanäle erstrecken sich in Axialrichtung des Fluidströmungskanals des Ventils und/oder Fluidströmungsrichtung.

In einer besonders bevorzugten Weiterbildung der Ventilinsel sind ein Teil der Vielzahl von Strömungskanälen, insbesondere acht, in unmittelbarer Nähe des Außenumfangs des Mischelements in Umfangsrichtung in gleichmäßigen Abständen angeordnet. Ein anderer Teil der Vielzahl von Strömungskanälen, insbesondere vier, sind in unmittelbarer Nähe der Mittelachse in Umfangsrichtung in gleichmäßigen Abständen angeordnet. Die Radialabstände des einen Teils der Vielzahl von Strömungskanälen zu der Mittelachse sind jeweils gleichmäßig. Ebenso sind die Radialabstände des anderen Teils der Vielzahl von Strömungskanälen zu der Mittelachse gleichmäßig.

In einer Weiterbildung der Ventilinsel ist die Querschnittsform des einen Teils der Vielzahl von Strömungskanälen eine andere als die Querschnittsform des anderen Teils der Vielzahl von Strömungskanälen. Der Querschnitt des einen Teils der Vielzahl von Strömungskanälen ist vorzugsweise kreisförmig ausgebildet. Der Querschnitt des anderen Teils der Vielzahl von Strömungskanälen ist vorzugsweise polygonal, insbesondere hexagonal, ausgebildet.

In einer Weiterbildung der Ventilinsel weist das Mischelement, insbesondere einen kegelstumpfförmigen, Kopplungsabschnitt auf, der in die Kopplungsaufnahme eingesetzt ist, und darin über ein Montageelement, insbesondere einem Montageclip, befestigt ist.

In einer Weiterbildung der Ventilinsel ist das Mischelement durch additive Fertigung oder Spritzgießen gefertigt.

In einer Weiterbildung der Ventilinsel ist das Mischelement einstückig mit dem Kopplungsabschnitt durch Spritzgießen oder additive Fertigung gefertigt. Das Mischelement kann als erste Komponente, beispielsweise als ein Vorspritzling, dienen, um anschließend mit einer zweiten Komponente, insbesondere dem Kopplungsabschnitt, durch Spritzgießen einstückig verbunden, insbesondere umspritzt, zu werden. Das Material der ersten Komponente und der zweiten Komponente kann unterschiedlich sein.

Durch den Einsatz des kegelstumpfförmigen Kopplungsabschnitts in die Kopplungsaufnahme wird eine fluidische, radialdichte Verbindung hergestellt. Mittels der fluidischen, radialdichten Verbindung werden die Fluidströmungskanäle des Grundkörpers und des Mischelements verbunden.

Das Mischelement kann direkt in eine Unterbaugruppe integriert sein, die beispielsweise ein Ventil, insbesondere Rückschlagventil, ein Montageclip und eine Leitfähigkeitsmesssensorik umfasst.

Das Mischelement kann mit einer Verdrehsicherung gegen ein Verdrehen um die Längs-/Mittelachse des Fluidströmungskanals des Ventils gesichert sein.

Ein weiterer Aspekt der Erfindung ist eine Hydraulikanordnung für eine oder einer extrakorporale(n) Blutbehandlungsmaschine mit einem Träger, an dem mittelbar oder unmittelbar zumindest eine Ventilinsel nach einem ersten Aspekt angeordnet ist.

Der Träger ist für einen modularen Einbau zumindest einer Komponente, aber vorzugsweise einer Mehrzahl von Komponenten, geeignet. Der Träger kann ein Turm, insbesondere Montageturm, sein.

In einer Weiterbildung der Hydraulikanordnung weist der Träger, insbesondere nur, eine Fläche oder Ebene auf, an der die eine oder die mehreren Ventilinseln und/oder andere Komponenten angeordnet ist oder sind. Dies hat den Vorteil, dass bei der der Wartung oder Reparatur der Hydraulikanordnung keine Komponenten den Weg versperren, die unter Umständen demontiert werden müssen, um an eine bestimmte Ventilinsel und/oder andere Komponente heranzukommen.

In einer Weiterbildung der Hydraulikanordnung ist die zumindest eine Ventilinsel und/oder andere Komponenten in der gemeinsamen Fläche oder Ebene bzw. Montageebene von einer gemeinsamen Seite aus montierbar oder demontierbar.

In einer Weiterbildung der Hydraulikanordnung weist der Träger zumindest einen angepassten Verbindungsabschnitt auf, der mit dem Verbindungsabschnitt des Grundkörpers, insbesondere werkzeuglos, verbunden ist.

Diese Verbindung kann insbesondere nach dem Schlüsselloch-Prinzip realisiert werden, bei dem ein zumindest männliches Verbindungselement in eine zumindest weibliche Verbindungsaufnahme eingesetzt (eingehängt) werden kann. Es sind aber auch andere werkzeuglose Verbindungsvarianten wie zum Beispiel ein Steckprinzip oder dergleichen möglich.

In einer Weiterbildung der Hydraulikanordnung weist diese einen Montageadapter mit einem angepassten Verbindungsabschnitt auf, der mit dem Verbindungsabschnitt des Grundkörpers, insbesondere werkzeuglos, verbunden ist, und zumindest einen sekundären Verbindungsabschnitt aufweist, der mit dem Träger verbunden ist.

Auch diese Verbindung kann insbesondere nach dem Schlüsselloch-Prinzip realisiert werden, bei dem ein zumindest männliches Verbindungselement in eine zumindest weibliche Verbindungsaufnahme eingesetzt (eingehängt) werden kann. Es sind aber auch andere werkzeuglose Verbindungsvarianten wie zum Beispiel ein Steckprinzip, Einhakprinzip oder dergleichen möglich, wie dies vorstehend bereits ausgeführt wurde. Der sekundäre Verbindungsabschnitt des Montageadapters kann eine kraftschlüssige oder werkzeuglose Verbindung mit dem Träger sein.

In einer Weiterbildung der Hydraulikanordnung ist der Montageadapter ein Distanzstück, über das der Grundkörper etwa parallel zur Fläche bzw. Ebene angeordnet ist, oder ein Montagewinkel, über den der Grundkörper abgewinkelt zur Fläche bzw. Ebene angeordnet ist.

In einer Weiterbildung der Hydraulikanordnung ist die Verbindung der Ventilinsel mit dem Träger oder dem Montageadapter, insbesondere über ein Arretierelement arretiert. Das Arretierelement kann ein Spreizclip, Keilstift oder dergleichen sein.

In einer Weiterbildung der Hydraulikanordnung ist der Träger ein Montageturm. Der Montageturm weist dabei eine Montageplatte oder dergleichen auf, die als Montagefläche bzw. Montageebene für die zumindest eine Ventilinsel und/oder andere hydraulische Komponenten dient. Die Montageplatte kann in einen U-förmigen Rahmen oder einem kastenförmigen Gehäuse vorgesehen sein. Die Montageplatte kann mittig und parallel zwischen der Gehäusevorder- und Rückwand angeordnet sein, so dass sich ein vorderer und hinterer Raum ausbildet, der über die Montageplatte abgegrenzt ist. An den gebildeten Innenflächen des jeweiligen Raums können weitere Ventilinseln bzw. andere hydraulische Komponenten, vorzugsweise werkzeuglos, montiert oder eingehängt werden.

In einer Weiterbildung der Hydraulikanordnung ist der Montagewinkel auf einem Einschubblech montiert, der zum Einführen bzw. Einschieben in die Hydraulikanordnung, insbesondere den Montageturm, geeignet ist.

In einer Weiterbildung der Hydraulikanordnung weist diese abweichend von der oder den Ventilinseln zumindest eine Unterbaugruppe auf, die an dem Träger mittelbar oder unmittelbar, insbesondere durch Einhängen und/oder Verschrauben, angeordnet ist.

Die Unterbaugruppe bildet dabei eine weitere Montagefläche oder -ebene, die planar zu der Montagefläche oder -ebene des Trägers ist. An der Montagefläche oder - ebene des Trägers kann zum Beispiel eine randseitig, flächige Aussparung vorgesehen sein, in welche die Unterbaugruppe planar eingesetzt ist oder wird.

In einer Weiterbildung der Hydraulikanordnung hat die Unterbaugruppe zumindest eine Bilanzierungskammer. Mit der Bilanzierungskammer können hydraulische Komponenten wie zum Beispiel Ventile, insbesondere Magnetventile, verbunden sein. Die Verbindung kann über ein Montageelement, insbesondere einem Montageclip, befestigt sein. Die Bilanzierungskammern können additiv oder mittels Spritzgießen gefertigt sein.

### Kurzbeschreibung der Figuren

Die oben angeführten Gegenstände, Aspekte und Vorteile der vorliegenden Erfindung werden durch die folgende detaillierte Beschreibung der zugehörigen Zeichnung weiter erläutert.
Fig. 1 ist eine schematische Darstellung einer Seite einer Ventilinsel gemäß einem ersten Ausführungsbeispiel der vorliegenden Offenbarung;
Fig. 2 ist eine schematische Darstellung der anderen Seite der Ventilinsel gemäß dem ersten Ausführungsbeispiel der vorliegenden Offenbarung;
Fig. 3 ist eine schematische Ansicht mehrerer Abwandlungen a) bis e) eines mit der Ventilinsel verbindbaren Steckventils, das einen Kopplungsabschnitt aufweist, der axial zu einem Spulenelement angeordnet ist;
Fig. 4 ist eine schematische Ansicht mehrerer Abwandlungen a) bis e) des mit der Ventilinsel verbindbaren Steckventils, das einen um 90° zu dem Spulenelement abgewinkelten Kopplungsabschnitt aufweist;
Fig. 5 ist eine schematische Darstellung der Einzelteile einer Konfiguration der Ventilinsel gemäß dem ersten Ausführungsbeispiel mit dem Steckventil, einem Montageclip, einem Spreizclip und einem Verbindungsabschnitt des Trägers;
Fig. 6 ist eine vergrößerte Darstellung einer Kopplungsaufnahme der Ventilinsel mit einem Montageclip gemäß dem ersten Ausführungsbeispiel;
Fig. 7 ist eine schematische Darstellung einer Vorderseite einer Ventilinsel gemäß einem zweiten Ausführungsbeispiel der vorliegenden Offenbarung;
Fig. 8 ist eine schematische Darstellung einer Montageseite einer Ventilinsel gemäß dem zweiten Ausführungsbeispiel der vorliegenden Offenbarung;
Fig. 9 ist eine schematische Darstellung der Einzelteile einer Konfiguration der Ventilinsel gemäß dem zweiten Ausführungsbeispiel mit Steckventilen, einem Spreizclip, einem Montageadapter und einem Verbindungsabschnitt des Trägers;
Fig. 10 ist eine schematische Darstellung einer Vorderansicht einer Gesamtkonfiguration des zweiten Ausführungsbeispiels der Ventilinsel;
Fig. 11 ist eine schematische Darstellung einer Hydraulikanordnung mit Bilanzierungskammern und der Ventilinsel gemäß dem zweiten Ausführungsbeispiel; und
Fig. 12 ist eine schematische Darstellung der Ventilinsel gemäß dem ersten Ausführungsbeispiel montiert an einem Montagewinkel, der an einem Einschubblech befestigt ist.
Fig. 13 ist eine schematische Darstellung der Einzelteile einer Konfiguration einer Unterbaugruppe mit einem Mischelement.
Fig. 14 ist eine schematische Darstellung der Einzelteile einer weiteren Konfiguration einer Unterbaugruppe mit einer Abwandlung des Mischelements.
Fig. 15 ist eine schematische Darstellung einer weiteren Abwandlung eines Mischelements.
Fig. 16 ist eine Querschnittsansicht der weiteren Abwandlung des Mischelements, das mit einem Kopplungsabschnitt einstückig verbunden ist.

### Ausführungsbeispiele

Nachstehend werden Ausführungsbeispiele der vorliegenden Offenbarung auf der Basis der zugehörigen Figuren beschrieben.

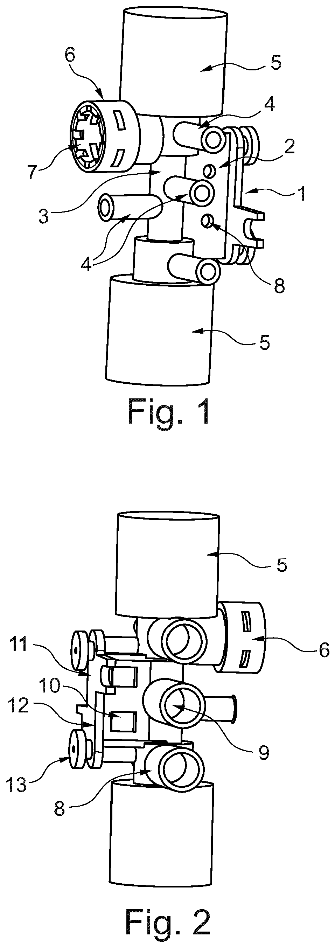

Die Fig. 1 zeigt eine schematische Darstellung einer Längsseite einer Ventilinsel 1 (Perspektivenansicht) gemäß einem ersten Ausführungsbeispiel der vorliegenden Offenbarung.

Die Ventilinsel 1 weist einen Grundkörper 2 auf. Der Grundkörper 2 ist mittels additiver Fertigung oder Spritzgießen gefertigt. Der Grundkörper 2 umfasst (hat) einen integralen/stoffeinstückig mit dem Grundkörper 2 ausgebildeten (innenliegenden, starren) Fluidströmungskanal 3, der somit eine selbstragende Funktion der Ventilinsel 1 erfüllt. Der Grundkörper 2 weist ferner eine Mehrzahl von integralen/ stoffeinstückig mit dem Grundkörper 2 ausgebildeten Tüllen oder Anschlussstutzen 4 auf, die mit dem Fluidströmungskanal 3 vorzugsweise T-förmig (fluid-)verbunden sind und zum Anschluss von externen hydraulischen Leitungen oder Schläuchen vorgesehenund ausgebildet sind. Die Tüllen 4 sind an dem Grundkörper 2 vorzugsweise angespritzt und weisen von dem Fluidströmungskanal 3 radial nach außen.

In/an dem Fluidströmungskanal 3 ist jeweils ein axialer Ventilsitz (nicht gezeigt) vorzugsweise innerhalb der beiden axialen Enden/Endabschnitte des Fluidströmungskanals 3 oder an den beiden axialen Enden/Endabschnitten des Fluidströmungskanals 3 vorgesehen oder ausgebildet. Jeder Ventilsitz wird von einem beweglichen Ventilelement (Ventilkolben) geöffnet und/oder geschlossen, das von einem am Grundkörper 2 bzw. Fluidströmungskanal 3 angeordneten Spulenelement (elektrische Ventilspule) 5 angesteuert wird. Die Spulenelemente 5 der beiden axial endseitig angeordneten Ventile befinden sich axial außerhalb an den axialen Enden/Endabschnitten des Fluidströmungskanals 3. Die Spulenelemente 5 sind in 45° Grad-Schritten oder alternativ in Schritten kleiner als 45° um ihre Achse, d.h. um die Längsachse des Fluidströmungskanals 3 drehbar am Grundkörper 2 gehalten.

Der Grundkörper 2 weist des Weiteren eine hohlzylinderförmige Kopplungsaufnahme (Port) 6 am/im Bereich des Ventilsitzes auf, die zur fluidischen und formschlüssigen (mechanischen) Verbindung mit anderen hydraulischen Komponenten geeignet ist. Die Kopplungsaufnahme 6 ist an den Grundkörper 2 vorzugsweise angespritzt und weist von dem Fluidströmungskanal 3 (vorzugsweise T-förmig) radial nach außen. Die hohlzylinderförmige Kopplungsaufnahme 6 weist innenumfangsseitig Vertiefungen 7 in vorzugsweise gleichen Umfangsabständen auf, die dafür geeignet und vorgesehen sind, ein darauf angepasstes Element einer anderen hydraulischen Komponente aufzunehmen. Der Grundkörper 2 weist zudem (Durchgangs-) Bohrungen 8 auf, die sich seitlich neben dem Fluidströmungskanal 3 durch den Grundkörper 2 vollständig hindurch erstrecken und durch die Fixiermittel wie Schrauben, Nieten oder Bänder (nicht gezeigt) einsetzbar sind.

In anderen Worten ausgedrückt, besteht die offenbarungsgemäße Ventilinsel aus dem Grundkörper 2, der sowohl den Fluidströmungskanal 3 wie auch einen (blockartigen) Abschnitt mit den darin ausgebildeten Bohrungen 8 stoffeinstückig aufweist. Der Fluidströmungskanal 3 hat zwei axialbeabstandete Enden/Endabschnitte, an denen die vorstehend genannten Magnetventile vorzugsweise um die KanalLängsachse drehbar montiert sind. Jedes Ventil hat eine elektrisch aktivierbare Ventilspule, mittels der jeweils ein beweglicher Ventilkörper für ein Öffnen und Schließen des Ventils bewegbar ist, um so den Fluidströmungskanal 3 mit einer Zweigleitung fluidzuverbinden und/oder fluidzutrennen. Der vom Ventilkörper öffen- und schließbare Ventilsitz ist entweder unmittelbar stirnseitig am Fluidströmungskanal 3 oder integral im Magnetventil ausgebildet. Alternativ zu der vorstehend beschriebenen Drehbarkeit der Ventil-internen Ventil-/Magnetspule kann diese (bzw. deren Aufnahmegehäuse) auch integral mit dem Grundkörper 2 ausgebildet sein.

Die Fig. 2 ist eine schematische Darstellung der bezüglich der Fig. 1 anderen Seite der Ventilinsel 1 gemäß dem ersten Ausführungsbeispiel der vorliegenden Offenbarung. An dieser anderen Seite des Grundkörpers 2 sind drei fluidische Verbindungsabschnitte 9 ausgebildet, die für die Verbindung/Ankopplung von, zum Beispiel, Sensoren oder Anzeigeinstrumenten geeignet sind. Die Verbindungsabschnitte 9 sind in Form von Anschlussstutzen dargestellt, die vorzugsweise T-förmig zum Fluidströmungskanal 3 ausgerichtet sind. Der Grundkörper 2 weist des Weiteren Steckerfenster 10 auf, die zur Aufnahme/Hindurchführung von Verkabelung oder des elektrischen Laststrangs für die Ventilspulen geeignet und vorgesehen sind und die neben dem Fluidströmungskanal 3 platziert sind.

Die in Fig. 2 gezeigte andere Seite der Ventilinsel 1 zeigt einen Verbindungsabschnitt oder Montageflansch 11 im Bereich des vorstehend genannten blockartigen Abschnitts des Grundkörpers 2, wobei der Verbindungsabschnitt/Montageflansch 11 an dem Grundkörper 2 stoffeinstückig ausgebildet ist. Der Verbindungabschnitt 11 ist dafür geeignet und vorgesehen, die Ventilinsel 1 mit einem darauf abgestimmten Verbindungsabschnitt (Montageflansch) einer Hydraulikanordnung oder eines Montageadapters (mechanisch) zu verbinden. Der Verbindungsabschnitt 11 besteht bevorzugt aus einer Verbindungsgrundplatte 12, die in Längsrichtung der Ventilinsel 1 parallel zu dem Fluidströmungskanal 3 des Grundkörpers 2 ausgerichtet ist. An den längs des Strömungskanals 3 beabstandeten Längsenden der Verbindungsgrundplatte 12 sind zwei männliche Verbindungselemente 13 (Positioniersockel/Einhängzapfen) ausgebildet, die zum Einsetzen oder Einhängen in jeweilige weibliche Verbindungsaufnahmen (Laschen/Bohrungen) zum Beispiel der Hydraulikanordnung geeignet und vorgesehen sind. Ein Steg des männliche Verbindungselements 13 kragt dabei aus der Verbindungsgrundplatte 12 aus, um in einem Kopfelement zu münden, das ein Vielfaches des Durchmessers des Stegs aufweist. Das Kopfelement spannt dabei eine Ebene parallel zu der Ebene der Verbindungsplatte 12 auf - ist also tellerförmig - und bildet so eine Hakenentsprechende Hinterschneidung.

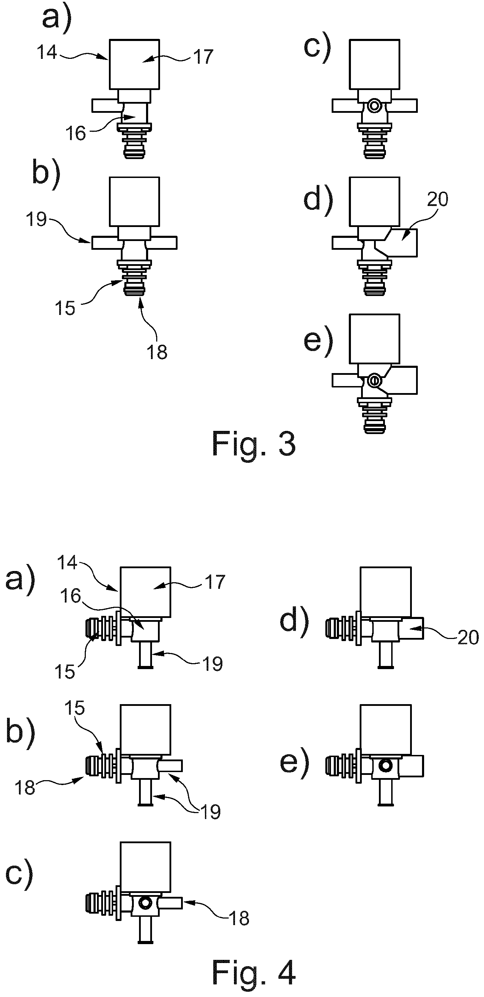

Die Fign. 3 und 4 zeigen ein Steckventil 14 in verschiedenen Konfigurationen, welches für den Einbau an der Kopplungsaufnahme (Port) 6 vorgesehen ist. Das Steckventil 14 ist vorzugsweise ein Magnetventil als Beispiel einer hydraulischen Komponente. Das Steckventil 14 ist dafür geeignet und vorgesehen, in die Kopplungsaufnahme 6 der Ventilinsel 1 über einen Kopplungsabschnitt 15 des Steckventils 14 eingesetzt zu werden, um mit dem Fluidströmungskanal 3 des Grundkörpers 2, radialdicht und formschlüssig verbunden zu sein. Das Steckventil 14 weist einen Ventilsitz 16 auf, der mittels eines beweglichen Ventilkörpers geöffnet und geschlossen werden kann, um so einen internen Fluidströmungskanal des Steckventils 14 zu öffnen und/oder zu sperren. Das Steckventil 14 weist des Weiteren, wie bei der Ventilinsel 1 gemäß Fig.1 und 2, ein elektrisches Spulenelement 17 auf.

Der Kopplungsabschnitt 15 des Steckventils 14 in Fig. 3 und 4 verjüngt sich kegelstumpförmig von einem Bodenabschnitt zu einem abgeflachten Spitzenabschnitt. In einer Axialrichtung (Längsrichtung) des Kopplungsabschnitt 15 hat dieser radiale Ausnehmungen, die radial nach innen bis zu einem äußeren Umfang des Fluidströmungskanals des Kopplungsabschnitts 15 reichen. Am Umfang des Spitzenabschnitts ist ein O-Ring 18 als Dichtelement angeordnet, der geeignet und vorgesehen ist, die Verbindung eines zur Ventilinsel 1 gebildeten Fluidströmungskanals radialdichtend auszubilden.

Die verschiedenen Konfigurationen des Steckventils 14 weisen eine oder eine Mehrzahl von Tüllen 19 auf, die mit dem internen Fluidströmungskanal des Steckventils 14 verbunden sind und zum Anschluss von externen hydraulischen Leitungen oder Schläuchen vorgesehen sind. Des Weiteren können einige der Konfigurationen des Steckventils 14 zusätzlich zu der einen oder der Mehrzahl von Tüllen 19 einen Verbindungsabschnitt 20 aufweisen, der für die Verbindung/Ankopplung von, zum Beispiel, Sensoren oder Anzeigeinstrumenten geeignet und vorgesehen ist.

Die Konfigurationen a) bis e) in Fig. 3 des Steckventils 14 haben einen Kopplungsabschnitt 15, der axial zu dem Spulenelement 17 ausgebildet ist. Die Konfigurationen des Steckventils 14 in Fig. 4 a) bis e) unterscheiden sich von denen der Fig. 3 im Wesentlichen dadurch, dass hier ein Kopplungsabschnitt 15 um 90° abgewinkelt zu dem Spulenelement 17 ausgebildet ist.

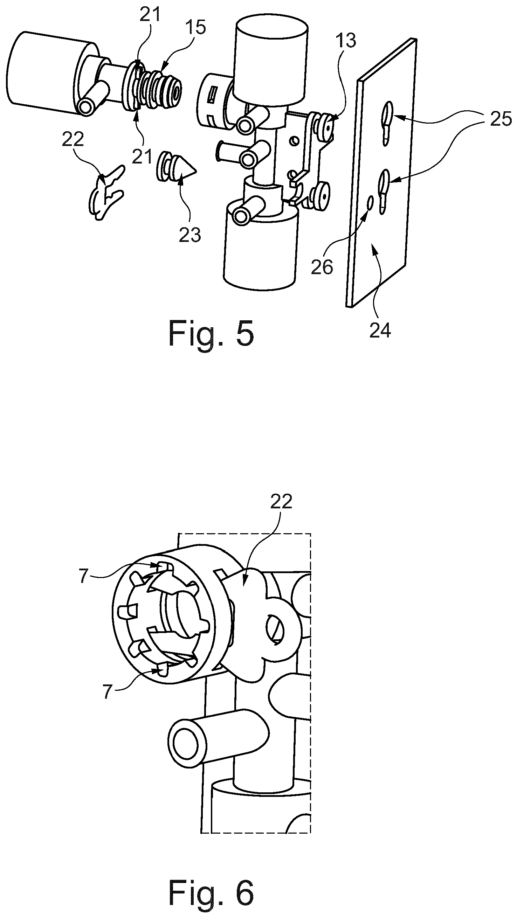

Fig. 5 ist eine schematische Darstellung der Einzelteile einer Konfiguration der Ventilinsel 1 gemäß dem ersten Ausführungsbeispiel (in einer Explosionsdarstellung) mit dem Steckventil 14, einem Montageclip 22 als Montage-/Axialsicherungselement, einem Ventilarretierelement 23 und einem Verbindungsabschnitt/Halteplatte 24 des Trägers/Ventilinsel-Halterung als Montageschnittstelle.

Ein Bodenabschnitt des Kopplungsabschnitts 15 des Steckventils 14 (entspricht einer Endscheibe, welche dem Kopplungsabschnitt 15 axial begrenzt), hat eine kreisförmige Stirnfläche, die in Axialrichtung hin zum Kopplungsabschnitt 15 weist und in deren Mitte der Fluidströmungskanal des Steckventils 14 mit einem kleineren Durchmesser als derjenige der kreisförmigen Stirnfläche angeordnet ist. An der kreisförmigen Stirnfläche sind zwei Vorsprünge 21 angeordnet, die in Axialrichtung von der kreisförmigen Stirnfläche in Richtung hin zum Kopplungsabschnitt 15 vorstehen und sich zudem in Radialrichtung, gemäß der Fig. 5 jeweils oberhalb und unterhalb des Fluidströmungskanals des Steckventils 14 nach jeweils oben und unten erstrecken. Im verbunden Zustand (Steckventil 14 ist in den Port 6 eingesetzt) greifen die Vorsprünge 21 am Kopplungsabschnitt 15 in die jeweilige Vertiefung 7 der Kopplungsaufnahme/Port 6 ein. Damit wird eine axiale Verdrehsicherung des Steckventils 14 im Verhältnis zu der Ventilinsel 1 realisiert.

Der Verbindungabschnitt 24 des Trägers weist zwei, gemäß der Fig. 5 mit Abstand übereinander angeordnete, weibliche Verbindungsaufnahmen 25 auf, die schlüssellochförmig ausgebildet sind und geeignet sowie vorgesehen sind, die jeweiligen zwei männlichen Verbindungselemente 13 der Ventilinsel 1 durch zum Beispiel Einhängen in die weiblichen Verbindungsaufnahmen 25 an dem Verbindungabschnitt 24 des Trägers zu befestigen.

Das Ventilarretierelement 23 ist kegelförmig ausgebildet und zum Arretieren der Ventilinsel 1 an dem Verbindungsabschnitt 24 des Trägers geeignet. Zum Arretieren der Ventilinsel 1 wird dabei das Ventilarretierelement 23 durch einen U-förmigen Abschnitt der Verbindungsgrundplatte 12 der Ventilinsel 1 (siehe Fig. 2), der seitlich zu der Längserstreckung der Verbindungsgrundplatte 12 aus dieser auskragt (siehe Fig. 5), und durch eine dafür vorgesehene Bohrung 26 am Träger eingesetzt/eingedrückt und durch radiale Spreizwirkung arretiert/verankert.

Die Fig. 6 zeigt eine vergrößerte Darstellung einer Kopplungsaufnahme 6 der Ventilinsel 1 mit einem Montageclip 22 gemäß dem ersten Ausführungsbeispiel. Der Montageclip 22 hat einen Basisabschnitt von dessen oberen und unteren Ende sich im 90°-Winkel jeweils ein Schenkel erstreckt, die zusammen mit dem Basisabschnitt eine U-Form bilden und parallel sind. Die Schenkel des Montageclips 22 werden zum Montieren des Steckventils 14 aus der Radialrichtung in Verriegelungsöffnungen der Kopplungsaufnahme 6 eingesetzt und umgreifen teilweise den Fluidströmungskanal des darin eingesetzten Kopplungsabschnitt 15 in den radialen Ausnehmungen. Der Kopplungsabschnitt 15 wird durch Federkraft der Schenkel des Montageclips 22 in der Axialrichtung gesichert.

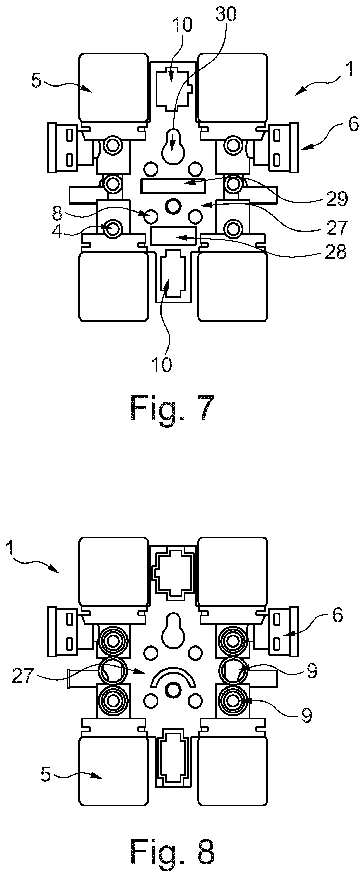

Die Fign. 7 und 8 zeigen eine schematische Darstellung einer Vorder- und einer Montageseite der Ventilinsel 1 gemäß einem zweiten Ausführungsbeispiel der vorliegenden Offenbarung.

Die Ventilinsel 1 des zweiten Ausführungsbeispiels unterscheidet sich von der Ventilinsel 1 des ersten Ausführungsbeispiels durch den Grundkörper 27, an dem ein weiterer (zweiter), unabhängiger Fluidströmungskanal ausgebildet ist, der vorzugsweise achsparallel zum ersten Fluidströmungskanal auf der gleichen oder einer gegenüberliegenden Grundkörperseite mit dem Grundkörper 27 stoffeinstückig verbunden ist. Mit anderen Worten dient der Grundkörper 27 u.a. als Mittel- bzw. Verbindungsteil zwischen den parallelbeabstandeten Fluidströmungskanälen.

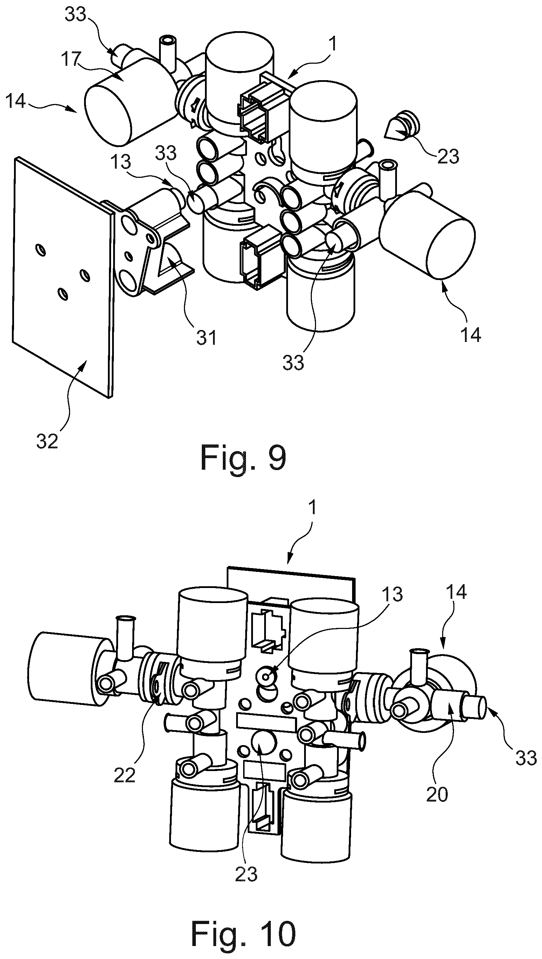

Die beiden Fluidströmungskanäle gemäß der Fign. 7 und 8 sind jeweils im Wesentlichen identisch zu dem Fluidströmungskanal 3 gemäß dem ersten Ausführungsbeispiel ausgebildet. D.h. jeder der beiden Fluidströmungskanäle des zweiten Ausführungsbeispiels hat zwei stirnseitig platzierte Spulenelemente 5 mit Ventilsitz, eine Kopplungsaufnahme/Port 6 für beispielsweise ein weiteres (Steck-) Ventil, eine Anzahl (vorzugsweise vier) Tüllen 4 für separate Leitungen und andere hydraulische Komponenten sowie eine Anzahl (vorzugsweise drei) fluidische Verbindungsabschnitten 9, die sämtlich stoffeinstückig mit den beiden Fluidströmungskanälen ausgebildet sind. Die jeweiligen Kopplungsaufnahmen 6 sind seitlich des Grundkörpers 27 angeordnet, derart, dass diese in entgegengesetzte Richtungen weisen und somit jeweils von seitlich außerhalb des Grundkörpers 27 zugänglich sind, ohne sich gegenseitig zu stören. Drei der Anzahl von Tüllen 4 sind auf einer (Vorder-)Seite und eine der Anzahl von Tüllen 4 ist seitlich (parallel zur jeweiligen Kopplungsaufnahme 6) des Grundkörpers 27 angeordnet. Die Anzahl von fluidischen Verbindungsabschnitte 9 sind sämtlich auf der Montageseite, d.h. auf der den Tüllen 4 abgewandten Längsseite der jeweiligen Fluidströmungskanäle angeordnet. Der Grundkörper 27 weist eine Anzahl, vorzugsweise vier Bohrungen 8 und eine Anzahl, vorzugsweise zwei Steckerfenster 10 auf. Die Vorderseite des Grundkörpers 27 ist mit einem Data-Matrix-Code 28 und einer Kennzeichnung für das Typenlabel 29 versehen. Der Grundkörper 27 weist eine ähnliche wie in dem ersten Ausführungsbeispiel beschriebene weibliche Verbindungsaufnahme 30 auf, die jedoch gedrehtschlüssellochförmig ausgebildet ist und geeignet und dafür vorgesehen ist, um in ein an einem zusätzlichen Montageadapter 31 (siehe Fig. 9) angeordneten männlichen Verbindungselement 13 zum Befestigen an dem Montageadapter 31 eingehängt zu werden, wobei der Montageadapter 31 wiederum zur Verbindung mit einer Halterung/Halteplatte/Träger 32 vorgesehen ist.

Die Fig. 9 zeigt eine schematische Darstellung der Einzelteile einer Konfiguration der Ventilinsel 1 des zweiten Ausführungsbeispiels mit zwei an den Kopplungsaufnahmen 6 (optional) angeschlossenen Steckventilen 14, dem Ventilarretierungselement (Spreizclip/- stift) 23, dem Montageadapter 31 und dem Träger bzw. dem Verbindungsabschnitt 32 des Trägers als Montageschnittstelle.

Der Montageadapter 31 dient in diesem Fall als Distanzstück, um einen Abstand zwischen der Ventilinsel 1 und dem Verbindungsabschnitt 32 des Trägers herzustellen. Dies ist aufgrund der besonderen Gestaltung der Montageseite (jene Seite, welche dem Träger zugewandt ist) der Ventilinsel 1 notwendig, auf der im zweiten Ausführungsbeispiel die fluidischen Verbindungsabschnitte 9 angeordnet sind. Ebenso wird der Abstand für das eine der beiden an der Kopplungsaufnahme 6 angeschlossene Streckventile benötigt, bei dem der Spulenkörper 17 des Steckventils um 90° zu dem Kopplungsabschnitt 15 des Steckventils in Richtung hin zum Träger abgewinkelt ist.

Der Montageadapter 31 weist auf einer der Ventilinsel zugewandten Seite zumindest eines von den zuvor beschriebenen männlichen Verbindungselementen (Haken) 13 auf, anders als bei der Ventilinsel 1 des ersten Ausführungsbeispiels, bei welcher die Verbindungsgrundplatte 12 des Grundkörpers 2 die männlichen Verbindungselemente 13 aufweist. Die dem Träger zugewandte Seite des Montageadapters 31 weist ferner eine Anzahl von (vorzugsweise drei) Bohrungen auf, die zur Montage an dem Träger geeignet sind, an dem deckungsgleich entsprechend gleichartige Bohrungen vorgesehen sind.

Fig. 10 zeigt eine schematische Darstellung einer Vorderansicht einer Gesamtkonfiguration der Ventilinsel 1 des zweiten Ausführungsbeispiels. In dieser Darstellung ist die Ventilinsel 1 über den Montageadapter 31 (in dieser Fig. nicht gezeigt) an dem Träger montiert und mittels des Spreizdorns 23 (siehe Fig. 5) arretiert. An den Kopplungsaufnahmen 6 des Grundkörpers 27 sind zwei Steckventile 14 angeschlossen/eingesetzt und mittels Montageclip/Splint 22 befestigt/axial fixiert. In den fluidischen Verbindungsabschnitten 20 des Steckventils 14 und einem der fluidischen Verbindungsabschnitte des Grundkörpers 27 ist eine Sensorik 33 optional eingesetzt.

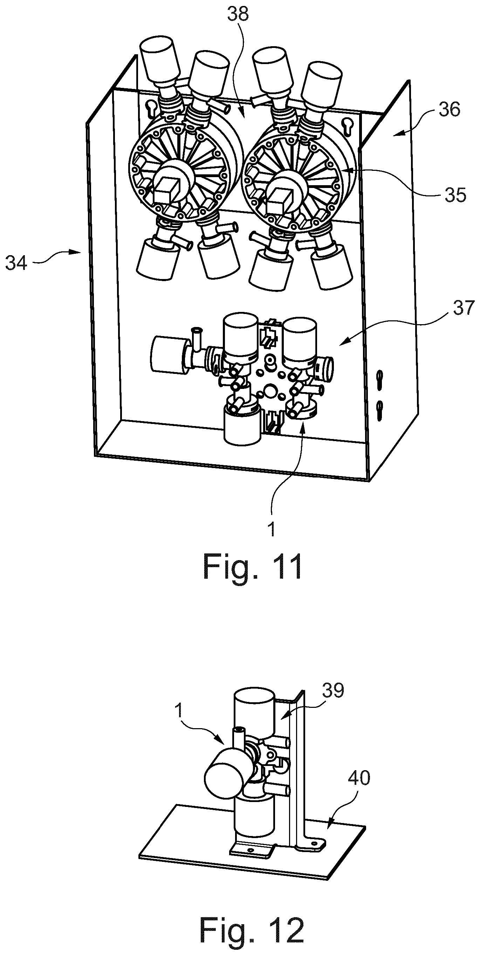

Fig. 11 ist eine schematische Darstellung eines Beispiels einer Hydraulikanordnung 34 in einer Dialysemaschine mit Bilanzierungskammern 35 und der Ventilinsel 1 gemäß dem zweiten Ausführungsbeispiel.

Ein Montageturm (Gehäuseabschnitt der Dialysemaschine) 36 weist bevorzugt eine Montageplatte 37 auf, die als Montagefläche oder Montageebene/Träger für die Ventilinsel 1 und ggf. auch für die Bilanzierungskammern 35 dient. Die Montageplatte 37 ist in einem U-förmigen Rahmen des Montageturms 36 vorgesehen. Der vom Montageturm 36 definierte/umgebene Raum wird durch die Montageplatte 37 in einen vorderen und einen hinteren Raumabschnitt geteilt. Die Ventilinsel 1 ist in einem Bereich, gemäß der Fig. 11, unter den Bilanzierungskammern 35 an die Montageplatte 37 montiert.

Die Bilanzierungskammern 35 sind auf einer plattenartigen Unterbaugruppe 38 befestigt, die in der Montageplatte 37 planar befestigt ist, um mit der Montageplatte 37 eine gemeinsame Ebene zu bilden.

Die Fig. 12 zeigt eine schematische Darstellung der Ventilinsel 1 gemäß dem ersten Ausführungsbeispiel, welche an einem Montagewinkel 39 montiert ist, der wiederum an einem Einschubblech 40 befestigt ist. Durch die Montage über den Montagewinkel 39 ist die Ventilinsel 1 gegenüber dem Einschubblech 40 um 90° abgewinkelt ausgerichtet. Das Einschubblech 40 ist zum Einführen bzw. Einschieben in die Hydraulikanordnung, zum Beispiel dem Montageturm vorzugsweise einer Dialysemaschine, geeignet und vorgesehen.

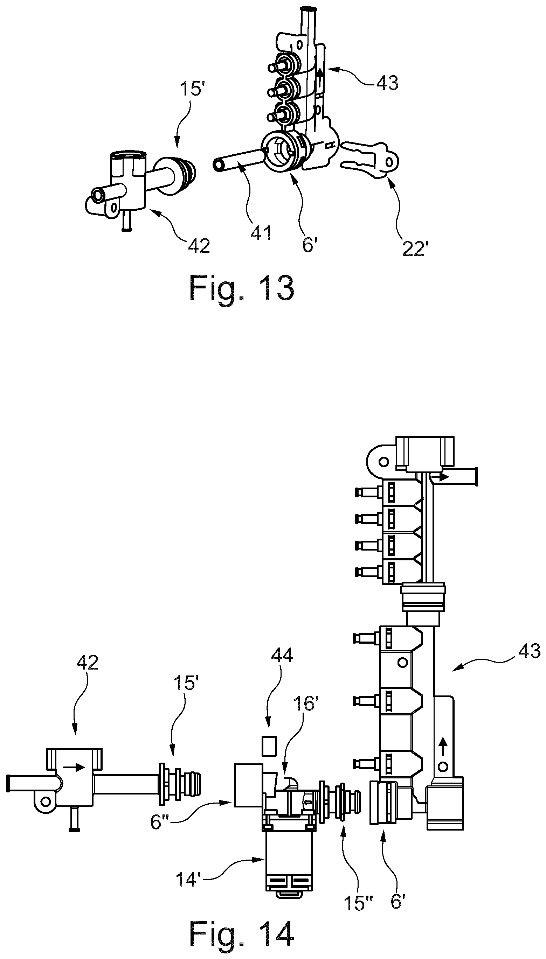

Die Fig. 13 zeigt eine schematische Darstellung der Einzelteile einer Konfiguration einer Unterbaugruppe mit einem Mischelement 41, das in einem Fluidströmungskanal eines Rückschlagventils 42 integriert ist. Das Rückschlagventil 42 mit dem integrierten Mischelement 41 ist dafür geeignet und vorgesehen, in eine Kopplungsaufnahme 6' der Unterbaugruppe oder in die Kopplungsaufnahme 6 der Ventilinsel 1 (siehe z.B. Fig. 1) über einen Kopplungsabschnitt 15' des Rückschlagventils 42 eingesetzt zu werden, um mit einem Fluidströmungskanal einer Leitfähigkeitsmesssensorik 43 oder der Ventilinsel 1 radialdicht und formschlüssig verbunden zu sein. Wie bereits in Fig. 6 gezeigt, ist die Verbindung dafür geeignet, mittels eines Montageclips 22' als Montageelement axial befestigt zu werden/sein.

Die Fig. 14 zeigt eine schematische Darstellung der Einzelteile einer weiteren Konfiguration einer Unterbaugruppe mit einem Mischelement 44, das in den Ventilsitz 16' eines Steckventils 14' integriert ist. Das Steckventil 14' mit dem integrierten Mischelement 44 ist dafür geeignet und vorgesehen, in eine Kopplungsaufnahme 6' der Unterbaugruppe oder in die Kopplungsaufnahme 6 der Ventilinsel 1 (siehe z.B. Fig. 1) über einen Kopplungsabschnitt 15" des Steckventils 14' eingesetzt zu werden, um mit einem Fluidströmungskanal einer Leitfähigkeitsmesssensorik 43 oder der Ventilinsel 1 radialdicht und formschlüssig verbunden zu sein. Das Rückschlagventil 42 (hier ohne Mischelement) ist dafür geeignet und vorgesehen, in eine Kopplungsaufnahme 6" des Steckventils 14' über den Kopplungsabschnitt 15' des Rückschlagventils 42 eingesetzt zu werden, um mit dem Steckventil 14' radialdicht und formschlüssig verbunden zu sein. In Fig. 14 ist somit folgende Verbindungsanordnung gezeigt: Rückschlagventil 42, Steckventil 14' mit integriertem Mischelement 41' und Leitfähigkeitsmesssensorik 43.

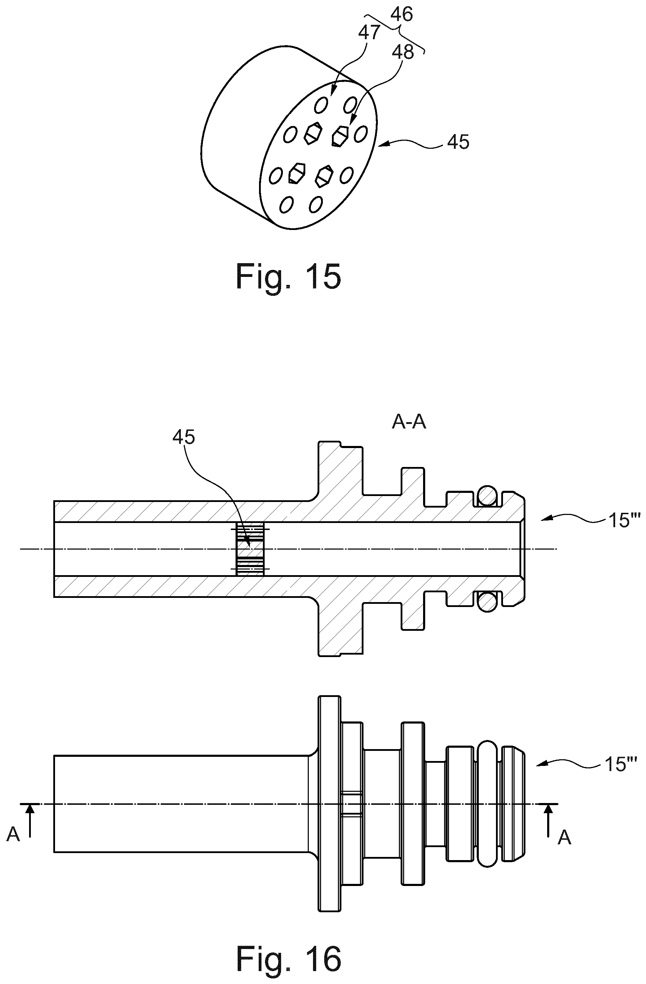

Die Fig. 15 zeigt eine schematische Darstellung einer weiteren Abwandlung eines Mischelements als Mischelement 45. Innerhalb des vollzylinderförmigen Mischelements 41" sind eine Vielzahl von Strömungskanälen 46 parallel zueinander und parallel zu einer Mittelachse A des Mischelements 45 angeordnet. Acht Strömungskanäle mit einem kreisförmigen Querschnitt 47 sind in unmittelbarer Nähe des Außenumfangs des Mischelements 45 in Umfangsrichtung in gleichmäßigen Abständen angeordnet. Vier Strömungskanäle mit einem hexagonalen Querschnitt 48 sind in unmittelbarer Nähe der Mittelachse A in Umfangsrichtung in gleichmäßigen Abständen angeordnet.

Die Fig. 16 zeigt eine Querschnittsansicht entlang einer Ebene A-A der Mittelachse A des Mischelements 45, das mit einem Kopplungsabschnitt 15‴ durch Spritzgießen oder additive Fertigung einstückig verbunden ist. Das Mischelement 41" kann als ein Vorspritzling mit dem Kopplungsabschnitt 15‴ umspritzt werden.

### Bezugszeichenliste

- 1: Ventilinsel
- 2: Grundkörper
- 3: Fluidströmungskanal
- 4: Tülle
- 5: Spulenelement, Ventilabschnitt
- 6, 6': Kopplungsaufnahme
- 7: Vertiefung
- 8: Bohrung
- 9: fluidischer/mechanischer Verbindungsabschnitt
- 10: Steckerfenster
- 11: Verbindungsabschnitt
- 12: Verbindungsgrundplatte
- 13: männliches Verbindungselement
- 14, 14': Steckventil
- 15, 15', 15", 15‴: Kopplungsabschnitt
- 16, 16': Ventilsitz des Steckventils
- 17: Spulenelement des Steckventils
- 18: O-Ring
- 19: Tülle des Steckventils
- 20: fluidischer/mechanischer Verbindungsabschnitt des Steckventils
- 21: Vorsprung
- 22, 22': Montageclip, Montageelement
- 23: Spreizclip/Ventilarretierungselement
- 24: Verbindungabschnitt des Trägers
- 25: weibliche Verbindungsaufnahme des Trägers
- 26: Bohrung des Trägers
- 27: Grundkörper
- 28: Data-Matrix-Code
- 29: Kennzeichnung Typenlabel
- 30: Verbindungsaufnahme, Verbindungabschnitt des Grundkörpers
- 31: Montageadapter
- 32: Verbindungsabschnitt des Trägers, sekundärer Verbindungsabschnitt
- 33: Sensorik
- 34: Hydraulikanordnung
- 35: Bilanzierungskammer
- 36: Montageturm
- 37: Montageplatte, Fläche
- 38: Unterbaugruppe
- 39: Montagewinkel
- 40: Einschubblech
- 41: Mischelement
- 42: Rückschlagventil
- 43: Leitfähigkeitsmesssensorik
- 44: Mischelement
- 45: Mischelement
- 46: Strömungskanäle des Mischelements
- 47: Strömungskanäle mit Kreisquerschnitt
- 48: Strömungskanäle mit hexagonalen Querschnitt

## Patentansprüche

1. Ventilinsel (1) für eine Hydraulikanordnung für eine extrakorporale Blutbehandlungsmaschine, vorzugsweise Dialysemaschine, mit einem Grundkörper (2, 27) an dem zumindest ein in Form eines Rohres ausgebildeter Fluidströmungskanal einstückig angeordnet ist, wobei der Grundkörper (2, 27) durch additive Fertigung oder Spritzgießen gefertigt ist,
**dadurch gekennzeichnet, dass** an zumindest einem axialen Ende des Fluidströmungskanals (3) ein Ventil angeordnet ist, über welches eine Fluidverbindung zwischen einer jeweiligen Fluidströmungskanalöffnung und einer ventilseitigen Zweigleitung öffenbar oder schließbar ist.

2. Ventilinsel (1) nach Anspruch 1, wobei einstückig mit dem Grundkörper (2, 27) zumindest ein Ventil-Montageabschnitt (5) ausgebildet ist.

3. Ventilinsel (1) nach Anspruch 2, wobei der Ventil-Montageabschnitt (5) in Schritten drehbar am Grundkörper (2, 27) angeordnet ist.

4. Ventilinsel (1) nach einem der vorhergehenden Ansprüche, wobei einstückig mit dem Grundkörper (2, 27) zumindest eine Kopplungsaufnahme (6) ausgebildet ist, über die eine hydraulische Komponente mit dem zumindest einen Fluidströmungskanal (3) fluidisch und formschlüssig verbindbar ist.

5. Ventilinsel (1) nach einem der vorhergehenden Ansprüche, wobei einstückig mit dem Grundkörper (2, 27) zumindest eine Tülle (4) ausgebildet ist, über die eine fluidische Leitung mit dem zumindest einen Fluidströmungskanal (3) fluidisch verbindbar ist.

6. Ventilinsel (1) nach einem der vorhergehenden Ansprüche, wobei einstückig mit dem Grundkörper (2, 27) zumindest ein fluidischer und/oder mechanischer Verbindungsabschnitt (9) ausgebildet ist, über den eine Komponente fluidisch und/oder mechanisch anbindbar ist.

7. Ventilinsel (1) nach einem der vorhergehenden Ansprüche, wobei am Grundkörper (2, 27) zumindest ein Verbindungsabschnitt (11, 30) ausgebildet ist, über den die Ventilinsel (1) mit einem darauf abgestimmten Verbindungsabschnitt des Trägers (24) oder eines Montageadapters (13) verbindbar ist.

8. Ventilinsel (1) nach Anspruch 4, wobei die hydraulische Komponente ein Ventil (14) ist, das über die Kopplungsaufnahme (6) mit dem zumindest einen Fluidströmungskanal (3) fluidisch und formschlüssig verbunden ist.

9. Ventilinsel (1) nach Anspruch 8, wobei das Ventil einen Kopplungsabschnitt (15) aufweist, der in die Kopplungsaufnahme (6) eingesetzt ist, und darin über ein Montageelement (22) befestigt ist.

10. Ventilinsel (1) nach Anspruch 8, wobei ein Mischelement (41) zum Mischen von Fluiden in einem Fluidströmungskanal des Ventils (14) oder eines Ventils (42, 14') integriert ist.

11. Ventilinsel (1) nach Anspruch 8, wobei ein Mischelement (44) in einem Ventilsitz des Ventils (14, 14') integriert ist.

12. Ventilinsel (1) nach Anspruch 4, wobei die hydraulische Komponente ein Mischelement (45) ist, das über die Kopplungsaufnahme (6) oder eine Kopplungsaufnahme (6', 6") mit einem Fluidströmungskanal oder dem zumindest einen Fluidströmungskanal (3) fluidisch und formschlüssig verbunden ist.

13. Ventilinsel (1) nach Anspruch 12, wobei das Mischelement (45) einen Kopplungsabschnitt (15‴) aufweist, der in die Kopplungsaufnahme (6, 6', 6") eingesetzt ist, und darin über ein Montageelement (22') befestigt ist.

14. Hydraulikanordnung für eine extrakorporale Blutbehandlungsmaschine mit einem Träger, an dem mittelbar oder unmittelbar zumindest eine Ventilinsel (1) nach einem der vorhergehenden Ansprüche angeordnet ist.

15. Hydraulikanordnung nach Anspruch 14, wobei der Träger eine Fläche (37) aufweist, an der die zumindest eine Ventilinsel (1) angeordnet ist oder sind.

16. Hydraulikanordnung nach Anspruch 14 oder 15, wobei die eine oder mehrere Ventilinseln (1) von einer gemeinsamen Seite aus montierbar oder demontierbar sind.

17. Hydraulikanordnung nach einem der Ansprüche 14 bis 16 mit der Ventilinsel (1), wobei der Träger zumindest einen angepassten Verbindungsabschnitt (24) hat, der mit einem Verbindungsabschnitt des Grundkörpers (11) verbunden ist.

18. Hydraulikanordnung nach einem der Ansprüche 14 bis 17 mit der Ventilinsel (1) und mit einem Montageadapter (31), wobei dieser zumindest einen angepassten Verbindungsabschnitt (13) hat, der mit dem Verbindungsabschnitt des Grundkörpers (30) verbunden ist, und zumindest einen sekundären Verbindungsabschnitt (32) hat, der mit dem Träger verbunden ist.

19. Dialysemaschine mit einer Hydraulikanordnung nach einem der Ansprüche 14 bis 18.

## Claims

1. A valve island (1) for a hydraulic assembly for an extracorporeal blood treatment machine, preferably a dialysis machine, with a base body (2, 27) on which at least one fluid flow passage (3) configured in the form of a tube is integrally arranged, wherein the base body (2, 27) is manufactured by additive manufacturing or injection molding,
**characterized in that**, at at least one axial end of the fluid flow passage (3), a valve (5) is arranged via which a fluid connection between a respective fluid flow channel opening and a valve-side branch line can be opened or closed.

2. The valve island (1) according to claim 1, wherein at least one valve mounting portion (5) is integrally formed with the base body (2, 27).

3. The valve island (1) according to claim 2, wherein the valve portion (5) is rotatably arranged on the base body (2, 27) in steps.

4. The valve island (1) according to one of the preceding claims, wherein at least one coupling receptacle (6) is formed integrally with the base body (2, 27), via which a hydraulic component can be connected fluidically and positively to the at least one fluid flow passage (3).

5. The valve island (1) according to one of the preceding claims, wherein at least one spout (4) is formed integrally with the base body (2, 27), via which a fluidic line can be fluidically connected to the at least one fluid flow passage (3).

6. The valve island (1) according to one of the preceding claims, wherein at least one fluidic and/or mechanical connecting portion (9) is formed integrally with the base body (2, 27), via which a component can be connected fluidically and/or mechanically.

7. The valve island (1) according to one of the preceding claims, wherein at least one connecting portion (11, 30) is formed on the base body (2, 27), via which the valve island (1) can be connected to a connecting portion of the carrier (24) or a mounting adapter (13) matched thereto.

8. The valve island (1) according to claim 4, wherein the hydraulic component is a valve (14) that is fluidically and positively connected to the at least one fluid flow passage (3) via the coupling receptacle (6).

9. The valve island (1) according to claim 8, wherein the valve comprises a coupling portion (15) inserted into the coupling receptacle (6) and secured therein via a mounting element (22).

10. The valve island (1) according to claim 8, wherein a mixing element (41) for mixing fluids is integrated in a fluid flow passage of the valve (14) or of a valve (42, 14').

11. The valve island (1) according to claim 8, wherein a mixing element (44) is integrated in a valve seat of the valve (14, 14').

12. The valve island (1) according to claim 4, wherein the hydraulic component is a mixing element (45) that is fluidically and positively connected to a fluid flow passage or to the at least one fluid flow passage (3) via the coupling receptacle (6) or a coupling receptacle (6', 6").

13. The valve island (1) according to claim 12, wherein the mixing element (45) comprises a coupling portion (15‴) inserted into the coupling receptacle (6, 6', 6") and secured therein via a mounting element (22').

14. A hydraulic assembly for an extracorporeal blood treatment machine with a carrier on which at least one valve island (1) according to one of the preceding claims is arranged indirectly or directly.

15. The hydraulic assembly according to claim 14, wherein the carrier includes a surface (37) on which the at least one valve island (1) is or are disposed.

16. The hydraulic assembly according to claim 14 or 15, wherein the one or more valve islands (1) are mountable or dismountable from a common side.

17. The hydraulic assembly according to any one of claims 14 to 16 comprising the valve island (1), wherein the carrier has at least one adapted connecting portion (24) connected to a connecting portion of the base body (11).

18. The hydraulic assembly according to one of claims 14 to 17, comprising the valve island (1) and a mounting adapter (31), wherein it has at least one adapted connecting portion (13) connected to the connecting portion of the base body (30) and at least one secondary connecting portion (32) connected to the carrier.

19. A dialysis machine comprising a hydraulic assembly according to one of claims 14 to 18.

## Revendications

1. Îlot de soupapes (1) pour un ensemble hydraulique pour une machine de traitement extracorporel du sang, de préférence une machine de dialyse, avec un corps de base (2, 27) au niveau duquel est agencé d'un seul tenant au moins un canal d'écoulement de fluide réalisé sous la forme d'un tube, dans lequel le corps de base (2, 27) est fabriqué par fabrication additive ou moulage par injection, **caractérisé en ce qu'**une soupape est agencée au niveau d'au moins une extrémité axiale du canal d'écoulement de fluide (3), soupape par le biais de laquelle une liaison fluidique peut être ouverte ou fermée entre une ouverture de canal d'écoulement de fluide respective et une conduite de dérivation côté soupape.

2. Îlot de soupapes (1) selon la revendication 1, dans lequel au moins une section de montage de soupape (5) est réalisée d'un seul tenant avec le corps de base (2, 27).

3. Îlot de soupapes (1) selon la revendication 2, dans lequel la section de montage de soupape (5) est agencée par étapes de manière rotative au niveau du corps de base (2, 27).

4. Îlot de soupapes (1) selon l'une quelconque des revendications précédentes, dans lequel au moins un logement de couplage (6) est réalisé d'un seul tenant avec le corps de base (2, 27) par le biais duquel un composant hydraulique peut être relié de manière fluidique et par complémentarité de formes avec l'au moins un canal d'écoulement de fluide (3).

5. Îlot de soupapes (1) selon l'une quelconque des revendications précédentes, dans lequel au moins un embout (4) est réalisé d'un seul tenant avec le corps de base (2, 27), embout par le biais duquel une conduite fluidique peut être reliée de manière fluidique à l'au moins un canal d'écoulement de fluide (3).

6. Îlot de soupapes (1) selon l'une quelconque des revendications précédentes, dans lequel au moins une section de liaison (9) fluidique et/ou mécanique est réalisée d'un seul tenant avec le corps de base (2, 27), section par le biais de laquelle un composant peut être relié de manière fluidique et/ou mécanique.

7. Îlot de soupapes (1) selon l'une quelconque des revendications précédentes, dans lequel au moins une section de liaison (11, 30) est réalisée au niveau du corps de base (2, 27), section par le biais de laquelle l'îlot de soupapes (1) peut être relié à une section de liaison adaptée du support (24) ou d'un adaptateur de montage (13).

8. Îlot de soupapes (1) selon la revendication 4, dans lequel le composant hydraulique est une soupape (14) qui est reliée de manière fluidique et par complémentarité de formes à l'au moins un canal d'écoulement de fluide (3) par le biais du logement de couplage (6).

9. Îlot de soupapes (1) selon la revendication 8, dans lequel la soupape présente une section de couplage (15) qui est insérée dans le logement de couplage (6) et est fixée dans celui-ci par le biais d'un élément de montage (22).

10. Îlot de soupapes (1) selon la revendication 8, dans lequel un élément de mélange (41) est intégré pour le mélange de fluides dans un canal d'écoulement de fluide de la soupape (14) ou d'une soupape (42, 14').

11. Îlot de soupapes (1) selon la revendication 8, dans lequel un élément de mélange (44) est intégré dans un siège de la soupape (14, 14').

12. Îlot de soupapes (1) selon la revendication 4, dans lequel le composant hydraulique est un élément de mélange (45) qui est relié de manière fluidique et par complémentarité de formes à un canal d'écoulement de fluide ou à l'au moins un canal d'écoulement de fluide (3) par le biais du logement de couplage (6) ou d'un logement de couplage (6', 6").

13. Îlot de soupapes (1) selon la revendication 12, dans lequel l'élément de mélange (45) présente une section de couplage (15‴) qui est insérée dans le logement de couplage (6, 6', 6") et est fixée dans celui-ci par le biais d'un élément de montage (22').

14. Ensemble hydraulique pour une machine de traitement extracorporel du sang avec un support au niveau duquel au moins un îlot de soupapes (1) selon l'une quelconque des revendications précédentes est agencé directement ou indirectement.

15. Ensemble hydraulique selon la revendication 14, dans lequel le support présente une surface (37) au niveau de laquelle l'au moins un îlot de soupapes (1) est agencé.

16. Ensemble hydraulique selon la revendication 14 ou 15, dans lequel les un ou plusieurs îlots de soupapes (1) peuvent être montés ou démontés depuis un côté commun.

17. Ensemble hydraulique selon l'une quelconque des revendications 14 à 16 avec l'îlot de soupapes (1), dans lequel le support présente au moins une section de liaison (24) adaptée qui est reliée à une section de liaison du corps de base (11).

18. Ensemble hydraulique selon l'une quelconque des revendications 14 à 17 avec l'îlot de soupapes (1) et avec un adaptateur de montage (31), dans lequel celui-ci présente au moins une section de liaison (13) adaptée qui est reliée à la section de liaison du corps de base (30) et présente au moins une section de liaison (32) secondaire qui est reliée au support.

19. Machine de dialyse avec un ensemble hydraulique selon l'une quelconque des revendications 14 à 18.
